# EUROPEAN PATENT SPECIFICATION

(11) **EP 0 304 986 B1**
(45) Date of publication and mention of the grant of the patent: **28.04.1993**
(21) Application number: 88201733.8
(22) Date of filing: 15.08.1988
(51) Int. Cl.: A61K 31/19, A23L 1/29, A23L 2/26

(54) **The use of certain Calcium-Citrate-Malate for the manufacture of a medicament for the treatment of osteoporosis**
Verwendung von bestimmten Calcium-Citrate-Malaten zur Herstellung einer pharmazeutischen Zubereitung für die Behandlung von Osteoporose.
Utilisation de certains Calcium-Citrate-Malate pour l'obtention d'un médicament destiné au traitement de l'ostéoporose

(30) Priority: 28.08.1987 US 91006
(43) Date of publication of application: 01.03.1989
(73) Proprietor: Norwich Eaton Pharmaceuticals, Inc., Norwich New York 13815 (US)
(72) Inventor: Kochanowski, Barbara Ann, West Chester Ohio 45069 (US)
(74) Representative: Brooks, Maxim Courtney

(56) References cited:
- EP-A- 0 297 679
- FR-A- 2 601 249
- CALCIFIED TISSUE INTERNATIONAL, vol. 4, no. 6, December 1987, pages 351-352, Springer Verlag, New York, US; K.T. SMITH et al.: "Calcium absorption from a new calcium delivery system (CCM)"
- CHEMICAL ABSTRACTS, vol. 96, no. 5, February 1, 1982, page 605, ref. 34574n, Columbus, Ohio, US
- THE NEW ENGLAND JOURNAL OF MED., vol. 323, no. 13, pages 878-883, Dawson-Hughes et al.

## Description

This invention relates to building bone in humans and other animals, i.e., to the treatment of osteoporosis. In particular, this invention relates to the administration of certain calcium salts.

Calcium is the fifth most abundant element in the human body. It plays an important role in many physiological processes, including nerve and muscle functions. Not surprisingly, nutritional and metabolic deficiencies of calcium can have broad-ranging adverse effects. Since about 90% of the body's calcium is found in bone tissues, many of these adverse effects are manifested through deficiencies in the structure, function and integrity of the skeletal system.

The most common metabolic bone disorder is osteoporosis. Osteoporosis can be generally defined as the reduction in the quantity of bone, or the atrophy of skeletal tissue. In general, there are two types of osteoporosis: primary and secondary. "Secondary osteoporosis" is the result of an identifiable disease process or agent. However, approximately 90% of all osteoporosis cases is idiopathic "primary osteoporosis". Such primary osteoporosis includes postmenopausal osteoporosis, age-associated osteoporosis (affecting a majority of individuals over the age of 70 to 80), and idiopathic osteoporosis affecting middle-aged and younger men and women.

For some osteoporotic individuals the loss of bone tissue is sufficiently great so as to cause mechanical failure of the bone structure. Bone fractures often occur, for example, in the wrist and spine of women suffering from postmenopausal osteoporosis. Kyphosis (abnormally increased curvature of the thoracic spine) may also result.

The mechanism of bone loss in osteoporotics is believed to involve an imbalance in the process of "bone remodeling". Bone remodeling occurs throughout life, renewing the skeleton and maintaining the strength of bone. This remodeling occurs in a series of discrete pockets of activity in the bone, called "osteoclasts" and "osteoblasts". Osteoclasts (bone dissolving or resorbing cells) are responsible for the resorption of a portion of bone within the bone matrix, during the resorption process. After resorption, the osteoclasts are followed by the appearance of osteoblasts (bone forming cells), which then refill the resorbed portion with new bone.

In a healthy adult, the rate at which the osteoclasts and osteoblasts are formed maintains a balance of bone resorption and bone formation. However, in osteoporotics an imbalance in this remodeling process develops, resulting in loss of bone at a rate faster than the accretion of bone. This imbalance is much more severe, and occurs at a younger age, in osteoporotics as compared to healthy adults.

Many compositions and methods are described in the medical literature for the "treatment" of osteoporosis. Many of these compositions and methods attempt to either slow the loss of bone or to produce a net gain in bone mass. See, for example, R. C. Haynes, Jr. et al., "Agents affecting Calcification", The Pharmacological Basis of Therapeutics, 7th Edition (A. G. Gilman, L. S. Goodman et al., Editors, 1985); and G. D. Whedon et al., "An Analysis of Current Concepts and Research Interest in Osteoporosis", Current Advances in Skeletogenesis (A. Ornoy et al., Editors, 1985). Estrogen is often used to affect the metabolism of calcium. Treatments using fluoride have also been described. However, the utility of such agents may be limited, due to possible adverse side effects. See W. A. Peck, et al., Physician's Resource Manual on Osteoporosis (1987), published by the National Osteoporosis Foundation.

Nutritional therapies for osteoporosis have also been proposed. Many calcium-containing compounds and compositions have been described for use as nutritional supplements. Many commercial preparations are also available, typically containing calcium carbonate. Calcium chloride, calcium gluceptate, calcium gluconate, calcium lactate, calcium phosphate, calcium citrate, and other calcium salts have also been described for use in calcium supplements. The use of calcium citrate, for example, is described in French Patent 2,219,778, Monteau, published September 27, 1974; and World Patent Publications 86/04814 and 86/04815, Pak et al., both published August 28, 1986. Food supplements containing calcium citrate malate are described in Japanese Patent Document 56/97, 248, Kawai, published August 5, 1981.

The utility of these known supplements varies. Unlike agents (such as estrogen) which affect the metabolism of bone, calcium nutritional supplements have been thought to merely provide a source for calcium (which may or may not be properly absorbed and metabolized). Indeed, the literature is bereft of any credible clinical data supporting the utility of any of these calcium supplements to actually treat osteoporosis (to actually build bone). See, for example, B. Riis et al., "Does Calcium Supplementation Prevent Postmenopausal Bone Loss?", 316 New England J. of Medicine 173-177 (1987); L. Nilas et al., "Calcium Supplementation and Postmenopausal Bone Loss", 289 British Medical Journal 1103-1106 (1984); and H. Spencer et al., "NIH Consensus Conference: Osteoporosis", 116 Journal of Nutrition 316-319 (1986).

It has now been discovered, however, that certain calcium citrate malate are surprisingly effective for the building of bone. In particular, as compared to nutritional regimens known in the art, these calcium citrate malates afford greater efficacy in the treatment of osteoporosis.

The present invention provides the use of certain calcium citrate malate for the manufacture of a medicament for the treatment of osteoporosis. The calcium citrate malate comprises a complex or a mixture of calcium salts having a ratio of moles citrate to moles malate of from 1:0.5 to 1:4.5. A preferred calcium citrate malate for use in the methods of this invention has a molar composition of calcium:citrate:malate of 6:2:3. The calcium citrate malate - comprising medicament is preferably administered in an oral dosage form, containing pharmaceutically-acceptable carriers and excipients.

### DESCRIPTION OF THE INVENTION

The present invention allows the administration of calcium citrate malate to a human or other animal subject. Specific compounds and compositions to be used in practising the present invention must, accordingly, be pharmaceutically-acceptable. As used herein, such a "pharmaceutically-acceptable" component is one that is suitable for use with humans and/or animals without undue adverse side effects (such as toxicity, irritation, and allergic response) commensurate with a reasonable benefit/risk ratio. Further, as used herein, the term "safe and effective amount" refers to the quantity of a component which is sufficient to yield a desired therapeutic response without undue adverse side effects (such as toxicity, irritation, or allergic response) commensurate with a reasonable benefit/risk ratio when used in the manner of this invention. The specific "safe and effective amount" will, obviously, vary with such factors as the particular condition being treated, the physical condition of the patient, the duration of the treatment, the nature of concurrent therapy (if any), and the specific formulations employed.

### Calcium Citrate Malate and Compositions:

The present invention involves the use of a mixture of calcium salts, herein "calcium citrate malate", comprising calcium salts of citric acid and malic acid for the manufacture of a medicament for the treatment of osteoporosis. The calcium citrate malate may consist of a mixture of calcium citrate and calcium malate, a complex of calcium containing citrate and malate ligands, a mixture of a calcium salt with citric acid and malic acid, or combinations thereof. (Mixtures of a calcium salt and citric and malic acids may be used to form calcium citrate malate in situ, in a liquid dose form, or in the acid environment of the stomach of the subject to whom the mixture is administered.)
The molar ratio of citrate:malate is from 1:0.5 to 1:4.5, preferably from 1:0.75 to 1:3. A preferred calcium citrate malate has a molar citrate:malate ratio of 1:1.5.

The ratio of moles calcium: total moles citrate plus malate is from 1:0.2 to 1:1.5, preferably from 1:0.7 to 1:0.9, more preferably 1:0.83. Accordingly, the calcium citrate malate may contain other acid anions in addition to citrate and malate. Such anions may include, for example, carbonate, hydroxide, and mixtures thereof.

Preferably, the calcium citrate malate is neutral, comprised entirely of citrate and malate anions. Thus, preferably, the equivalents of calcium (2 x moles calcium) is about equal to the total number of equivalents of citrate (3 x moles citrate) plus malate (2 x moles malate). A preferred calcium citrate malate has a calcium:citrate:malate molar composition of 6:2:3. Such a preferred calcium citrate malate is described in copending EP Patent Application EP 304 987, Jacobs, "Novel Calcium Supplements", filed August 28, 1987.

The calcium citrate malate for use in this invention may be provided in solid or liquid dosage forms. Calcium citrate malate for use in solid forms may be made, for example, by first dissolving citric acid and malic acid, in the desired molar ratio, in water. Calcium carbonate may then be added to the solution, in such amount that the ratio of moles calcium to moles citrate and moles malate is as desired. Carbon dioxide will be evolved. The solution may then be dried (as by freeze drying or oven drying) to obtain the calcium citrate malate. Methods for making calcium citrate malate are described in the following documents: European Patent Publication 208,362, Anastasia et al., published January 14, 1987; Japanese Patent Specification SHO 56-97248, Kawai, published August 5, 1981; and U.S. Patent Application Serial No. 860,607, Heckert, "Fruit Juice Beverages and Juice Concentrates Nutritionally Supplemented with Calcium", filed May 7, 1986.

Various oral dosage forms of calcium citrate malate may be used in the present invention. Such dosage forms comprise a safe and effective amount of calcium citrate malate and a pharmaceutically-acceptable carrier. Preferably the pharmaceutically-acceptable carrier is present at a level of from 0.1% to 99%, preferably from 0.1% to 75%, by weight of the composition. Unit dosage forms (i.e., dosage forms containing an amount of calcium citrate malate suitable for administration in one single dose, according to sound medical practice) preferably contain from 100 mg (milligrams) to 1000 mg, preferably from 100 mg to 500 mg, more preferably from 200 mg to 300 mg of calcium (on an elemental basis).

Solid forms include tablets, capsules, granules and bulk powders. Aside from the calcium citrate malate, tablets may contain suitable binders, lubricants, diluents, disintegrating agents, coloring agents, flavoring agents, flow-inducing agents and melting agents. Liquid oral dosage forms include aqueous solutions, emulsions, suspensions, solutions and/or suspensions reconstituted from non-effervescent granules and effervescent preparations reconstituted from effervescent granules. Such liquid oral dosage forms may contain, for example, suitable solvents, preservatives, emulsifying agents, suspending agents, diluents, sweeteners, melting agents, and coloring and flavoring agents. A preferred liquid dosage form contains calcium citrate malate in a juice-containing beverage.

Specific examples of pharmaceutically-acceptable carriers and excipients that may be used to formulate oral dosage forms of the present invention are described in U. S. Patent 3,903,297, Robert, issued September 2, 1975. Techniques and compositions for making dosage forms useful in practising this invention are described in the following references: 7 Modern Pharmaceutics, Chapters 9 and 10 (Banker & Rhodes, Editors, 1979); Lieberman et al., Pharmaceutical Dosage Forms: Tablets (1981); and Ansel, Introduction to Pharmaceutical Dosage Forms 2d Edition. (1976).

### Methods of Treatment:

Specifically, the present invention allows the building of bone in a human or other animal subject, by administering to said subject a safe and effective amount of calcium citrate malate for a period of time sufficient to achieve an increase in the net skeletal mass of said subject. As used herein, "building bone" refers to an increase in the net skeletal mass of the subject treated. The increase in mass may be in cortical bone, trabecular bone, or both. Preferably, the net skeletal mass is increased by at least about 0.5%, more preferably at least about 1%. "Administering" refers to any method which, in sound medical practice, delivers the calcium citrate malate used in this invention to the subject to be treated in such a manner so as to be effective in the building of bone. Preferably, the calcium citrate malate is administered orally.

Preferably, from about 175 milligrams to about 2000 milligrams of calcium (as elemental calcium) are administered to said subject, per day. More preferably, from about 250 milligrams to about 1500 milligrams, more preferably from about 500 milligrams to about 1000 milligrams, of calcium are administered, per day. The specific amount of calcium citrate malate to be administered depends upon the relative percentage weight of calcium in the particular calcium citrate malate employed.

The specific period of time sufficient to achieve an increase in the net skeletal mass of the subject may depend on a variety of factors. Such factors include, for example, the specific calcium citrate malate formulation employed, the amount of calcium citrate malate administered, the age and sex of the subject, the specific disorder to be treated, concomitant therapies employed (if any), the general physical health of the subject (including the presence of other disorders), the extent of bone loss in the individual, and the nutritional habits of the individual. Although the administration of even small quantities of calcium citrate malate may build bone, the net increase in bone mass may not be detectable for short periods of administration.

For the treatment of osteoporosis, the calcium citrate malate is preferably administered for at least about three months, preferably for at least about six months. Of course, such administration may be continued indefinitely, according to sound medical practice. Preferably the subject is treated until a net skeletal mass is obtained that is clinically determined to be above the fracture threshold for the subject. See, B.L. Riggs et al., "Involutional Osteoporosis" 314 New England J. of Medicine (1986).

The present invention is for the treatment of osteoporosis, and may include administration of calcium citrate malate alone, or in combination with other therapeutic agents. In particular, in one embodiment of this invention calcium citrate malate is used for the manufacture of medicaments to be used in an "ADFR" regimen. Such a regimen, in general, comprises administration to the subject of a bone-cell activating agent (such as an inorganic phosphate); followed by administration of an osteoclast depressant, to inhibit bone resorption (such as a diphosphonate); followed by a "free" period during which osteoblast bone formation occurs. The entire cycle is preferably repeated. Such regimens, among those useful herein, are described in Belgian Patent Publication 902,307, Anderson et al., "Treatment of Osteoporosis", published October 29, 1985 and Belgian Patent Publication 902,308, Flora, "Treatment of Osteoporosis", published October 29, 1985. Preferably, in such regimens calcium citrate malate is administered during the free period. Kits to facilitate ADFR regimens are described in European Patent Specification 162,510, Uchtman, "Kit for Use in the Treatment of Osteoporosis", published November 27, 1985.

In another embodiment of this invention calcium citrate malate is used for the manufacture of medicaments to be used in a regimen comprising intermittent dosing of certain polyphosphonate compounds. Such methods comprise administration of the polyphosphonate followed by a "rest period". Such regimens, among those useful herein, are described in European Patent Specification 210,728, Flora et al., "Regimen for Treating Osteoporosis", published February 4, 1987. Preferably, calcium citrate malate is administered during the rest period.

### EXAMPLE I

A composition is prepared containing calcium citrate malate having a molar calcium:citrate:malate composition of about 6:2:3. The calcium citrate malate is made by first dissolving 384.2 grams of citric acid and 402.3 grams of malic acid in 2 liters of water. This citrate/malate solution is then heated to 55°C (131°F), with stirring. Separately, 600.6 grams of calcium carbonate is added to 1.2 liters of water, forming a slurry, with stirring.

The citrate/malate solution is then removed from its heat source, and the calcium carbonate slurry is added slowly, with stirring. The rate of addition is controlled, to contain the reaction as carbon dioxide is released. An additional quantity of water, 0.4 liters, is finally added. The reaction mixture is then stirred for 1 to 1.5 hours. The reaction is essentially complete as the pH of the solution equilibrates to 4.3.

A precipitate of calcium citrate malate is thus formed. The excess reaction liquid is filtered off. The calcium citrate malate is dried, for 12 hours at 105°C (221°F), reducing the moisture level to less than 1%. The dried product is then milled to 10-20 mesh size, for a swallowable tablet formulation.

The swallowable tablet dosage form is then made, comprising:

| Component | % (by weight) |
|---|---|
| calcium citrate malate* | 99.73 |
| magnesium stearate | 0.27 |

| | |
|---|---|
| *: having a molar calcium:citrate:malate composition of 6:2:3, made as described above in this Example. | |

The tablet formulation is made by thoroughly admixing the powders, and tabletting using a standard tablet press, to form tablets weighing 1104 milligrams. The tablets are then coated, using a pan coater. The coating solution contains 11% hydroxypropylmethyl cellulose, 2% polyethylene glycol, 3.5% colorant, and the balance of water.

The mass of the subject's thoracic vertebrae is determined by dual-energy photon absorptiometry. The human subject is then administered 4 of the tablets, comprised as above, each day for three months. The mass of the subject's vertebrae is then remeasured, indicating an increase in bone mass.

### EXAMPLE II

A beverage composition is prepared containing calcium citrate malate. The beverage is made comprising:

| Component | % (by weight) |
|---|---|
| 65° Brix Orange Juice Concentrate | 38.010 |
| aqueous orange essences | 10.099 |
| orange pulp | 4.958 |
| orange oils | 0.037 |
| orange flavor mix | 0.257 |
| calcium carbonate | 1.065 |
| citric acid | 1.249 |
| malic acid | 0.992 |
| sucrose | 16.710 |
| water | 26.623 |

A premix solution is prepared by dissolving the sugar and then the acids (citric and malic) in the water. Calcium carbonate is added and the mixture agitated until foaming ceases. This premix solution has a calcium to citrate/malate molar ratio of 1:1.31, and a citric acid:malic acid molar ratio of 1:1.14. The premix solution is added, with stirring, to the 65° Brix orange juice concentrate, followed by the orange essences, orange pulp, orange oil, and orange flavor mix. The resulting calcium-supplemented orange concentrate nectar has a sugar content of 42° Brix, 0.44% by weight calcium, calcium to citrate/malate molar ratio of 1:2.38, and a citric acid:malic acid molar ratio of 1:0.71.

This concentrated beverage is then diluted, one part concentrate with three parts water, to form a single strength orange nectar beverage in drinkable form. When diluted, the beverage contains 60% orange juice and 0.11% by weight calcium.

The density of the human subject's vertebrae is measured by computerized tomography. Thereafter, the subject is administered approximately 180 milliliters (6 ounces) of the beverage composition (containing approximately 195 milligrams of calcium), per day, for one year. The vertebral mass of the subject is then remeasured, indicating an increase in bone density.

### EXAMPLE III

A chewable tablet composition comprises as set forth below.

| Component | % (by weight) |
|---|---|
| calcium citrate malate* | 46.94 |
| mannitol | 46.69 |
| magnesium stearate | 0.68 |
| flavorant | 5.69 |

| | |
|---|---|
| * having a molar calcium:citrate:malate composition of 5:2:2, made in a manner analogous to that described in Example I, above. | |

The tablets are made by thoroughly admixing the powders, and tabletting on a standard tablet press, forming tablets weighing 1844 milligrams. Each tablet contains 250 mg of calcium (on an elemental basis).

The bone density of the subject's hip is measured by dual-energy photon absorptiometry. The subject is then administered 2 of the tablets, comprised as above, each day for six months. The bone density of the subject's hip is then remeasured, indicating an increase in the bone mass.

## Claims

1. The use of calcium citrate malate for the manufacture of a medicament for the treatment of osteoporosis wherein said calcium citrate malate has a molar ratio of citrate:malate of from 1:0.5 to 1:4.5.

2. The use according to claim 1, wherein said calcium citrate malate has a molar ratio of citrate:malate of from 1:0.75 to 1:3.

3. The use according to claim 1, wherein said calcium citrate malate has a molar ratio of calcium:total moles citrate plus malate of from 1:0.7 to 1:0.9.

4. The use according to claim 3, wherein the equivalents of calcium in said calcium citrate malate is equal to the total number of equivalents of citrate plus the equivalents of malate in said calcium citrate malate.

5. The use according to any of the preceding claims, wherein said calcium citrate malate-comprising medicament is in a solid dosage form.

6. The use according to any of the preceding claims, wherein said calcium citrate malate-comprising medicament is in a liquid dosage form.

7. The use according to claim 6, wherein the said liquid dosage form is a juice-containing beverage.

8. The use according to any of the preceding claims, wherein said medicament is in the form of a unit dose comprising from 100 mg to 1000 mg of calcium citrate malate.

## Patentansprüche

1. Verwendung von Calciumcitratmalat zur Herstellung eines Arzneimittels für die Behandlung von Osteoporose, wobei dieses Calciumcitratmalat ein Molverhältnis von Citrat:Malat von 1:0,5 bis 1:4,5 aufweist.

2. Verwendung nach Anspruch 1, wobei das Calciumcitratmalat ein Molverhältnis von Citrat:Malat von 1:0,75 bis 1:3 aufweist.

3. Verwendung nach Anspruch 1, wobei das Calciumcitratmalat ein Molverhältnis von Calcium:Gesamtmolen Citrat plus Malat von 1:0,7 bis 1:0,9 aufweist.

4. Verwendung nach Anspruch 3, wobei die Calcium-Äquivalente in dem genannten Calciumcitratmalat gleich der Gesamtanzahl von Citrat-Äquivalenten plus Malat-Äquivalenten in dem genannten Calciumcitratmalat sind.

5. Verwendung nach einem der vorstehenden Ansprüche, wobei das das Calciumcitratmalat enthaltende Arzneimittel in einer festen Dosierungsform vorliegt.

6. Verwendung nach einem der vorstehenden Ansprüche, wobei das das Calciumcitratmalat enthaltende Arzneimittel in einer flüssigen Dosierungsform vorliegt.

7. Verwendung nach Anspruch 6, wobei die flüssige Dosierungsform ein safthältiges Getränk ist.

8. Verwendung nach einem der vorstehenden Ansprüche, wobei das Arzneimittel in der Form einer Einheitsdosis vorliegt, welche 100 mg bis 1.000 mg an Calciumcitratmalat enthält.

## Revendications

1. Utilisation de citrate et malate de calcium pour préparer un médicament destiné au traitement de l'ostéoporose, dans laquelle ledit citrate et malate de calcium présente une proportion molaire du citrate au malate de 1:0,5 à 1:4,5.

2. Utilisation selon la revendication 1, dans laquelle ledit citrate et malate de calcium présente une proportion molaire du citrate au malate de 1:0,75 à 1:3.

3. Utilisation selon la revendication 1, dans laquelle ledit citrate et malate de calcium présente une proportion molaire du calcium au total du citrate et du malate de 1:0,7 à 1:0,9.

4. Utilisation selon la revendication 3, dans laquelle le nombre d'équivalents de calcium dans ledit citrate et malate de calcium est égal au nombre total d'équivalents de citrate augmenté du nombre d'équivalents de malate dans ledit citrate et malate de calcium.

5. Utilisation selon l'une quelconque des revendications précédentes, dans laquelle ledit médicament contenant du citrate et malate de calcium se présente sous une forme posologique solide.

6. Utilisation selon l'une quelconque des revendications précédentes, dans laquelle ledit médicament contenant du citrate et malate de calcium se présente sous une forme posologique liquide.

7. Utilisation selon la revendication 6, dans laquelle ladite forme posologique liquide est une boisson contenant un jus de fruits.

8. Utilisation selon l'une quelconque des revendications précédentes, dans laquelle ledit médicament se présente sous la forme d'une dose unitaire comprenant de 100 à 1000 mg de citrate et malate de calcium.
